# EUROPEAN PATENT APPLICATION

(11) **EP 4 567 429 A1**
(43) Date of publication of application: **11.06.2025**
(21) Application number: 23849729.1
(22) Date of filing: 23.05.2023
(51) Int. Cl.: G01N 35/00, G16H 10/40

(54) **ANALYZING DEVICE, AND INFORMATION OUTPUT METHOD**

(30) Priority: 05.08.2022 JP 2022125217
(71) Applicant: HITACHI HIGH-TECH CORPORATION, Tokyo 105-6409 (JP)
(72) Inventor: ITO Masayuki, Tokyo 105-6409 (JP)
(74) Representative: Strehl & Partner mbB
(86) International application number: PCT/JP2023/019184
(87) International publication number: WO 2024/029167

(57) **Abstract**

[Problem]

Provided is an analyzer that outputs information that makes it possible to trace a database and a log.

[Solution]

The analyzer includes: an analyzing unit that analyzes a sample; a storage unit that stores databases DB1 to DB3 in which a sample ID linked to personal information of a patient having supplied the sample, and sample information of the sample to be analyzed by the analyzing unit, an analysis result of the sample by the analyzing unit, or the personal information of the patient are stored in a corresponding manner and, a log including the sample ID; and a control unit that sets a unique identifier for each record of the database DB1, replaces the sample ID of the log with the identifier set for the sample ID of the database DB1 which is the same as the sample ID of the log (S1108), and after deleting the sample ID and personal information of the databases DB1 to DB3 and the log (S1112), outputs the databases DB1 to DB3 and the log to an external device (S1113).

## Description

### Technical Field

The present disclosure relates to an analyzer and an information output method, and relates to an analyzer and an information output method that output information to an external device.

### Background Art

An analyzer accumulates analysis results obtained by measuring a sample in a database together with personal information. The analyzer is connected to a host computer (clinical test information system) for the purpose of centralized control of sample information, and outputs a log including the sample information.

The log includes personal information of a patient who is a provider of a sample, and when a database or a log is output to an external device for the purpose of investigation (for example, investigation of mix-ups of samples, investigation of malfunctions of an automatic analyzer, or the like), all personal information serving as key information of investigation and related information linked to the personal information are hidden and output.

PTL 1 discloses a technique in which, when a serviceman who performs maintenance on a sample testing device outputs test results, attribute information by which a subject can be identified is hidden and output so that the subject is not identified.

### Citation List

### Patent Literature

PTL 1: JP2014-062917A

### Summary of Invention

### Technical Problem

In recent years, more strict information security control has been required. The control of personal information regarding medical products is also becoming stricter according to the EU General Data Protection Regulation (GDPR). When a database or a log is taken out to the outside for the purpose of an investigation performed by a serviceman due to an abnormality in an analysis result or the like, all information including personal information (for example, a name or a birth date) and related information (for example, information such as a sample ID or a patient ID) linked to the personal information must be deleted (hidden).

However, the personal information and the related information are often used as key information for investigation, and therefore, if the personal information and the related information are deleted (hidden) when the personal information and the related information are taken out to the outside, the database and the log cannot be traced, a problem location is hardly identified, and the investigation takes time.

The present disclosure has been made in view of the above problems, and an object thereof is to provide an analyzer and an information output method that output information by which a database and a log can be traced.

### Solution to Problem

An analyzer according to the present disclosure includes: an analyzing unit that analyzes a sample; a storage unit that stores a database in which related information linked to personal information of a patient having supplied the sample, and sample information of the sample to be analyzed by the analyzing unit, an analysis result of the sample by the analyzing unit, or the personal information of the patient are stored in a corresponding manner and a log including the related information; and a control unit that sets a unique identifier for each record of the database, sets, for the related information of the log, the identifier set for the related information of the database which is the same as the related information of the log, and after deleting the related information of the database and the related information of the log, outputs the database and the log to an external device.

### Advantageous Effects of Invention

According to the analyzer of the present disclosure, a database and a log can be traced using an identifier set in the database and an identifier set in the log.

Other technical problems and novel features will become apparent from description of the present description and the accompanying drawings.

### Brief Description of Drawings

Fig. 1 is a general configuration diagram of a multi-item chemical analyzer (automatic analyzer) according to an embodiment and a system connected to it.
Fig. 2 is a view of the composition of a sample control screen in a comparative example.
Fig. 3 is a view that shows a database of sample information in the comparative example.
Fig. 4 is a view that shows a database of analysis results in the comparative example.
Fig. 5 is a view of the composition of a patient information screen in the comparative example.
Fig. 6 is a view that shows a database of patient information in the comparative example.
Fig. 7 is a flowchart that shows the sequence for external output of databases and a log in the comparative example.
Fig. 8A is a view that shows databases in which personal information and related information linked to personal information are deleted, in the comparative example.
Fig. 8B is a view that shows logs in the comparative example.
Fig. 9A is a flowchart that shows the sequence for addition of sample information according to a first embodiment.
Fig. 9B is a view that shows a database for control of identifiers according to the first embodiment.
Fig. 9C is a view that shows a database of sample information to which identifiers are added, according to the first embodiment.
Fig. 9D is a view that shows a database for accumulation according to the first embodiment.
Fig. 10A is a flowchart that shows the sequence for deletion of sample information according to the first embodiment.
Fig. 10B is a view that shows a database before deletion of sample information according to the first embodiment.
Fig. 10C is a view that shows a database after deletion of sample information according to the first embodiment.
Fig. 10D is a view that shows a database for accumulation after deletion of sample information according to the first embodiment.
Fig. 11 is a view that shows a database to which screen output information is added according to a variation of the first embodiment.
Fig. 12 is a flowchart that shows the output method for databases and a log according to the first embodiment.
Fig. 13A is view that shows a database for control of identifiers in which identification information is combined according to a second embodiment.
Fig. 13B is view that shows a database for control of identifiers in which type information is combined according to the second embodiment.
Fig. 14 is view that shows a database for control of identifiers in which a device ID is combined according to the second embodiment.
Fig. 15A is a view that shows a sample control screen in the user mode and a database for reference according to a third embodiment.
Fig. 15B is a view that shows a sample control screen in the service mode and a database for reference according to the third embodiment.
Fig. 16 is a view that shows a database for control of log-in IDs in a database according to the third embodiment.
Fig. 17 is a view of the composition of a sample control screen according to the third embodiment.

### Description of Embodiments

In the following embodiments, when necessary for convenience, the description will be made by being divided into a plurality of sections or embodiments, but unless otherwise stated, they are not unrelated to each other, and one has a relation with all or a part of modifications, details, supplementary explanations, and the like of the other.

In the following embodiments, when referring to the number of elements (including the number, numerical values, amounts, ranges, or the like) or the like, the number of elements is not limited to a specific number, and may be the specific number or more or the specific number or less, unless otherwise specified or except a case where the number is apparently limited to a specific number in principle.

Further, in the following embodiments, it is needless to mention that components (also including element steps and the like) thereof are not necessarily essential unless otherwise specified or unless clearly considered to be essential in principle.

Similarly, in the following embodiments, when a shape, a positional relation, or the like of a component or the like is referred to, the shape or the like is substantially approximate or similar to the shape or the like unless otherwise specified or clearly considered otherwise in principle. The same applies to the above numerical values and ranges.

In all drawings illustrating the embodiments, the same members are denoted by the same reference numerals in principle, and repeated description thereof will be omitted.

In this description, the term "serviceman" means a person who goes to a facility where an analyzer is provided, and performs maintenance, inspection, and repair of the analyzer. In addition, in this description, "personal information" includes information by which a specific individual among information on individuals (patients) can be identified. In this description, "related information" is information linked to personal information.

It should be noted that the analyzer according to the invention is not limited to a multi-item chemical analyzer, and can be applied to various analyzers that handle patient samples.

### <Multi-Item Chemical Analyzer 100>

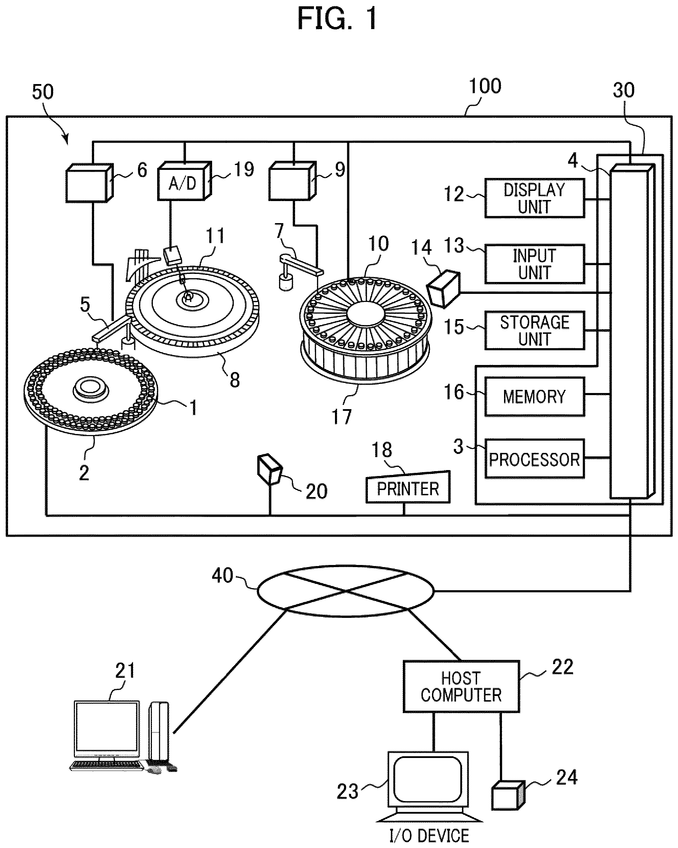

First, a configuration of a multi-item chemical analyzer 100 according to an embodiment will be described. Fig. 1 is an overall configuration diagram of the multi-item chemical analyzer 100 according to the embodiment and a system connected thereto. The multi-item chemical analyzer 100 mainly includes an analyzing unit 50 that analyzes a sample, a storage unit 15 that stores various types of information, and a control unit 30 that controls the operation of each unit of the multi-item chemical analyzer 100.

The analyzing unit 50 includes a sample disk 2, a reaction disk 8, a reagent disk 17, and peripheral devices thereof. The reaction disk 8 can be rotated intermittently, and a large number of reaction containers 11 made of a transparent material are arranged on the reaction disk 8 along a circumferential direction. Each of the reaction containers 11 is maintained at a predetermined temperature (for example, 37°C) by a constant-temperature bath. A large number of sample containers 1 that accommodate biological samples such as blood and urine are placed on the sample disk 2 in a double manner along a circumferential direction in the shown example. Further, a sample dispensing mechanism 5 is provided near the sample disk 2. The sample dispensing mechanism 5 includes a movable arm and a pipette nozzle attached to the movable arm. With the above configuration, when dispensing a sample, the pipette nozzle of the sample dispensing mechanism 5 moves to an dispensing position, aspirates a predetermined amount of sample from the sample container 1 located at an aspiration position on the sample disk 2, and discharges the aspirated sample into the reaction container 11 located at a discharging position on the reaction disk 8. The operation of the sample dispensing mechanism 5 is performed while detecting a liquid level of the sample container 1 or the reaction container 11 by a liquid level detector 6.

A plurality of reagent containers 10 to each of which a label showing reagent identification information, such as a barcode, adheres are placed on the reagent disk 17 along a circumferential direction of the reagent disk 17. The reagent container 10 accommodates a reagent liquid corresponding to an analysis item to be analyzed by the multi-item chemical analyzer 100. Further, a reagent ID reader 14 is attached to read a barcode shown on an outer wall of each reagent container 10 during reagent entry. The read reagent information is entered in a memory 16 or the storage unit 15 together with a position on the reagent disk 17.

A reagent dispensing mechanism 7 having substantially the same mechanism as the sample dispensing mechanism 5 is provided near the reagent disk 17. When dispensing a reagent, a pipette nozzle provided in the reagent dispensing mechanism 7 aspirates a reagent liquid from the reagent container 10 corresponding to a test item of the reaction container 11 positioned at a reagent receiving position on the reaction disk 8, and discharges the reagent liquid to the corresponding reaction container 11. The operation of the reagent dispensing mechanism 7 is also performed while detecting a liquid level by a liquid level detector 9.

A light source is provided near a center portion of the reaction disk 8 and a photometer is provided on an outer circumferential side of the reaction disk 8 to form a photodetection system. The row of the reaction containers 11 rotates and moves to pass through a photometric position sandwiched between the light source and the photometer, and a reaction liquid including the sample and the reagent in each reaction container 11 is subjected to photometry every time the reaction container passes in front of the photometer during rotation operation of the reaction disk 8. An analog signal of transmitted light or scattered light measured for each sample is converted into a digital signal by an A/D converter 19. An interior of the reaction container 11 for which the photometry has been completed is cleaned by a reaction container cleaning mechanism provided near the reaction disk 8, thereby enabling repeated use of the reaction container 11.

The control unit 30 not only controls an operation of each mechanism of the multi-item chemical analyzer 100 as described above, but also executes analysis of a sample according to the test item by receiving a detection signal from the photometer, which has been converted into a digital signal by the A/D converter 19. The control unit 30 is a computer system and includes a processor 3, the memory 16, and an interface 4. The control unit 30 has a hardware structure of a general information processing device (computer system). That is, a program code of software stored in the storage unit 15 such as a hard disk drive (HDD) or a solid state drive (SSD) is read into the memory 16 (main memory), and the processor 3 executes the read program code to execute information processing. A display unit 12, an input unit 13, the storage unit 15, a printer 18, and a reader 20 are communicably connected to the control unit 30 via the interface 4. The storage unit 15 stores databases DB1 to DB7 and logs described below.

The multi-item chemical analyzer 100 is communicably connected to a host computer (clinical test information system) 22 provided in a consultation room or the like, and is capable of transmitting and receiving information via a network 40. The host computer 22 is communicably connected to an I/O device 23 including a display unit and the like and a reader 24. The host computer 22 is also connected to an information control system 21 provided at a hospital reception desk or the like and is capable of transmitting and receiving information via the network 40. The information control system 21 and the host computer 22 each have a hardware structure of a general information processing device (computer system). That is, the program code of the software stored in the storage unit is read into the main memory, and the processor executes the read program code to execute the information processing.

### (Comparative Example)

Here, a comparative example will be described.

### <Sample Control Screen 200 of Comparative Example>

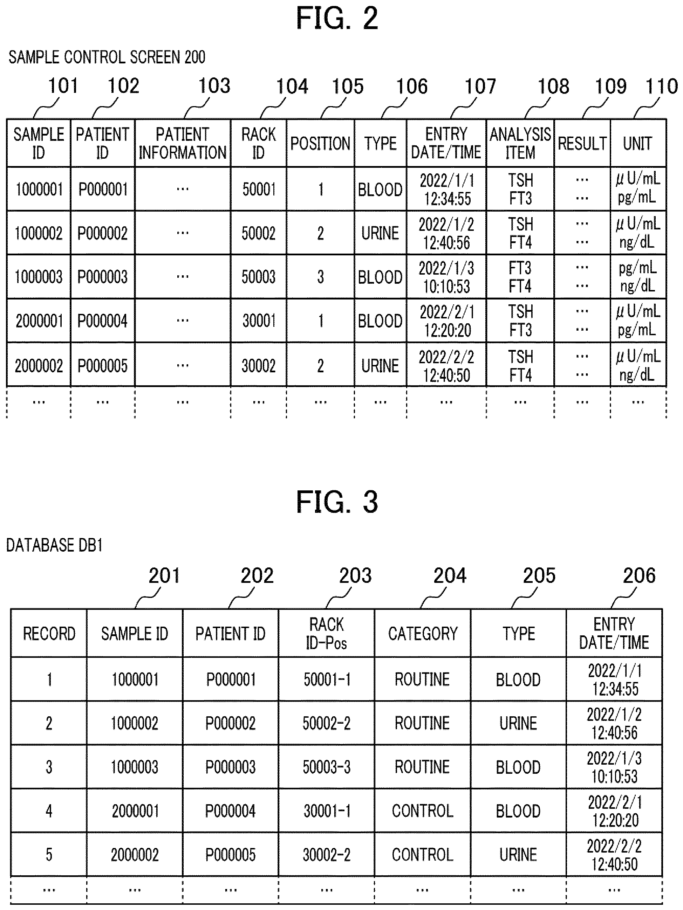

First, the sample control screen 200 of Comparative Example will be described. Fig. 2 is a configuration diagram of the sample control screen 200 of the comparative example. As shown in Fig. 2, the sample control screen 200 of the comparative example is a screen including an area 101 for displaying a sample ID, an area 102 for displaying a patient ID, an area 103 for displaying details of patient information, an area 104 for displaying a rack ID, an area 105 for displaying a position in a rack, an area 106 for displaying sample type information, an area 107 for displaying an entry date/time of a sample, an area 108 for displaying an analysis item requested for the sample, an area 109 for displaying an analysis result, and an area 110 for displaying a unit of an analysis result.

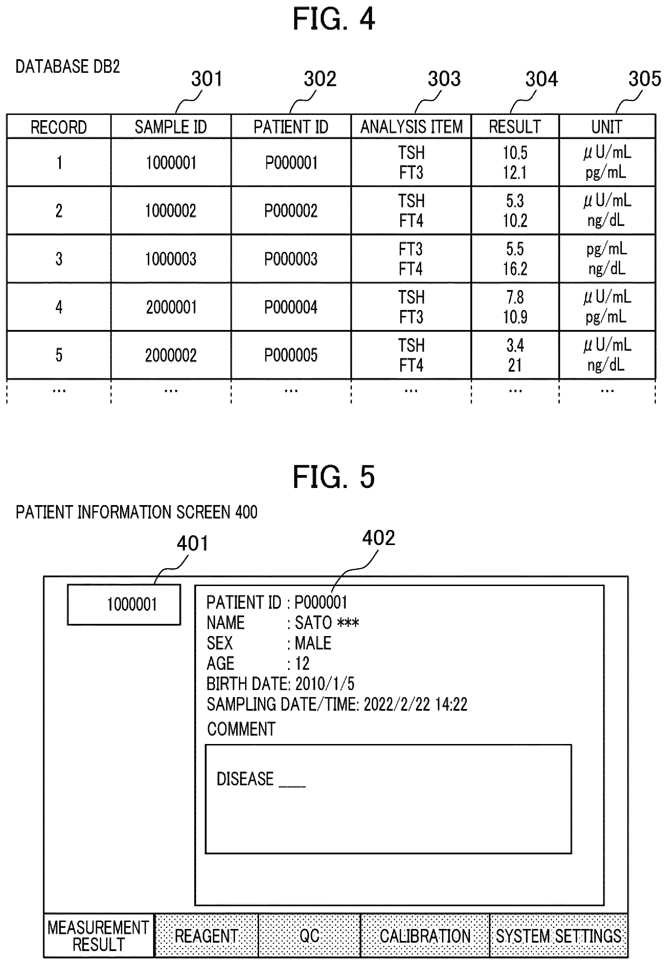

The sample control screen 200 is updated when entry of a sample is performed to execute analysis or when the analysis result is checked. The sample control screen 200 displays related information (for example, a sample ID or a patient ID) linked to the personal information. In addition, when accessing details in the area 103, a patient information screen 400 (Fig. 5) displaying personal information such as a name and a birth date is displayed. The control unit 30 displays the sample control screen 200 on the display unit 12 by referring to the database DB1 (Fig. 3) in which the related information and the sample information are stored in association with each other and the database DB2 (Fig. 4) in which the related information and the analysis result of the sample are stored in a corresponding manner.

### <Database DB1 of Sample Information in Comparative Example>

The database DB1 in the comparative example in Fig. 3 is a database that stores related information (for example, a sample ID or a patient ID) linked to personal information of a patient who provides a sample and sample information (for example, a rack ID, identification information, type information, and entry date/time) of a sample analyzed by the analyzing unit 50 in a corresponding manner. The database DB1 has a plurality of records, and each record has a sample ID field 201, a patient ID field 202, a rack ID-Pos (information including a rack ID and a position in a rack) field 203, a sample identification information field 204, a sample type information field 205, and a sample entry date/time field 206. That is, the database DB1 stores the sample ID, the patient ID, the rack ID-Pos, the sample identification information, the sample type information, the sample entry date/time, and the like in a corresponding manner.

### <Database DB2 of Analysis Results of Comparative Example>

The database DB2 in the comparative example in Fig. 4 is a database that stores related information (for example, a sample ID or a patient ID) linked to personal information of a patient who provides a sample and analysis results (for example, an analysis item, an analysis result, and a unit) of the sample obtained by the analyzing unit 50 in a corresponding manner. The database DB2 has a plurality of records, and each record has a sample ID field 301, a patient ID field 302, an analysis item field 303, an analysis result field 304, and an analysis result unit field 305. That is, the database DB2 stores the sample ID, the patient ID, the analysis item, the analysis results, the unit of the analysis results, and the like in a corresponding manner.

### <Patient Information Screen 400 of Comparative Example>

Fig. 5 is a configuration diagram of the patient information screen 400 of the comparative example. The patient information screen 400 is displayed by selecting details displayed in the area 103 shown in Fig. 2. As shown in Fig. 5, the patient information screen 400 is a screen having an area 401 for displaying a sample ID, and an area 402 of a patient information and the like for displaying a patient ID, a name, a sex, an age, a birth date, a sampling date/time, and a comment.

The patient information screen 400 is a screen in which any information can be input. The patient information screen 400 has information including personal information. The control unit 30 displays the patient information screen 400 on the display unit 12 by referring to the database DB3 in which related information and patient information described below are stored in a corresponding manner.

### <Database DB3 of Patient Information of Comparative Example>

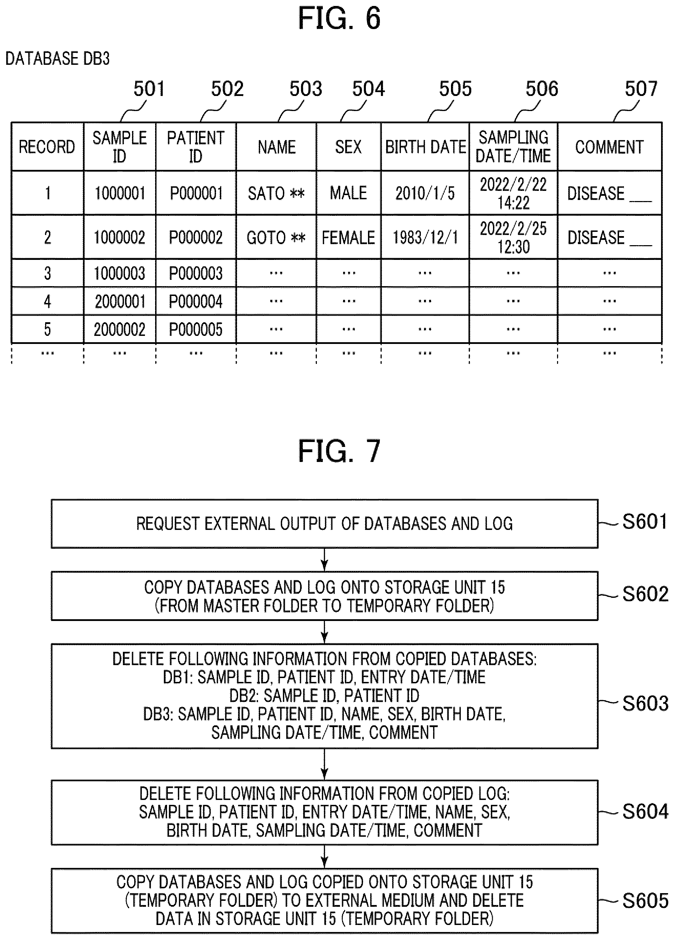

The database DB3 of the comparative example in Fig. 6 is a database that stores related information (for example, a sample ID and a patient ID) linked to personal information of a patient who provides a sample and personal information (for example, a name, a sex, a birth date, a sampling date/time, and a comment) of the patient in a corresponding manner. The database DB3 has a plurality of records, and each record has a sample ID field 501, a patient ID field 502, a name field 503, a sex field 504, a birth date field 505, a sampling date/time field 506, and a comment field 507. That is, the database DB1 stores the sample ID, the patient ID, the name, the sex, the birth date, the sampling date/time, the comment, and the like in association with one another.

### <External Output of Databases and Logs (Comparative Example)>

When the multi-item chemical analyzer 100 and the host computer 22 are connected, the control unit 30 stores, in the storage unit 15, logs including the sample ID, the patient ID, the rack ID, the position, the type information, and the entry date/time included in the database DB1 of sample information, the sample ID, the patient ID, the analysis item, the analysis results, and the unit included in the database DB2 of analysis results, and the sample ID, the patient ID, the name, the sex, the birth date, the sampling date/time, and the comment included in the database DB3 of patient information.

The multi-item chemical analyzer 100 has a function to be capable of storing the databases DB1 to DB3 and the logs in an external medium by connecting the external medium (external device) such as USB or DVD media to the input unit 13.

When storing the databases DB1 to DB3 and the logs in the external medium, the control unit 30 executes the processing according to the flowchart in Fig. 7. A method of outputting the databases DB1 to DB3 and the logs to the external medium (information output method) will be described with reference to Fig. 7.

When the external output of the databases and the logs is requested (step S601), the control unit 30 copies the databases DB1 to DB3 and the logs from a master folder in the storage unit 15 in which the databases DB1 to DB3 and the logs are stored to a temporary folder of the storage unit 15 (step S602). Then, the control unit 30 deletes personal information (for example, the name, the sex, the birth date, the sampling date/time, and the comment) and related information (for example, the sample ID, the patient ID, and the entry date/time) linked to the personal information from the copied databases DB1 to DB3 and the logs (steps S603 and S604). Finally, the control unit 30 copies the databases DB1 to DB3 and the logs copied to the temporary folder of the storage unit 15 to the external medium, and deletes the data in the temporary folder of the storage unit 15 (step S605). Hereinafter, "personal information and information linked to the personal information" are appropriately referred to as "personal information and the like".

### <Database of Temporary Folder from which Personal Information and the like are Deleted>

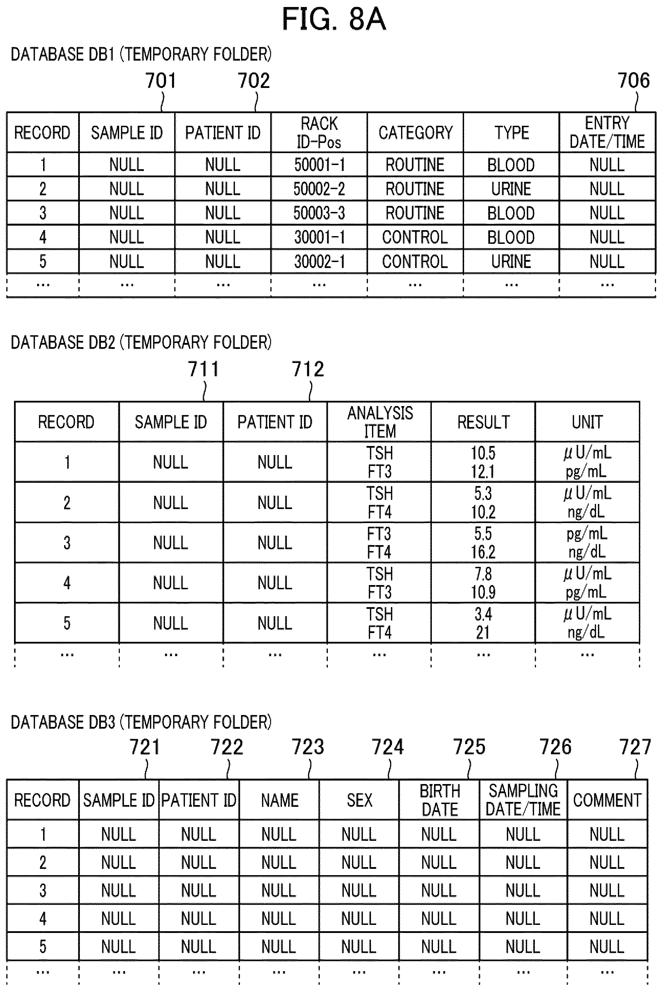

As shown in Fig. 8A, in the database DB1 of the temporary folder from which the personal information and the like are deleted, a sample ID field 701, a patient ID field 702, and an entry date/time field 706 are fields to be deleted. In the database DB2 of the temporary folder from which the personal information and the like are deleted, a sample ID field 711 and a patient ID field 712 are fields to be deleted. In the database DB3 of the temporary folder from which the personal information and the like are deleted, a sample ID field 721, a patient ID field 722, a name field 723, a sex field 724, a birth date field 725, a sampling date/time field 726, and a comment field 727 are fields to be deleted.

### <Logs in Temporary Folder from which Personal Information and the like are Deleted>

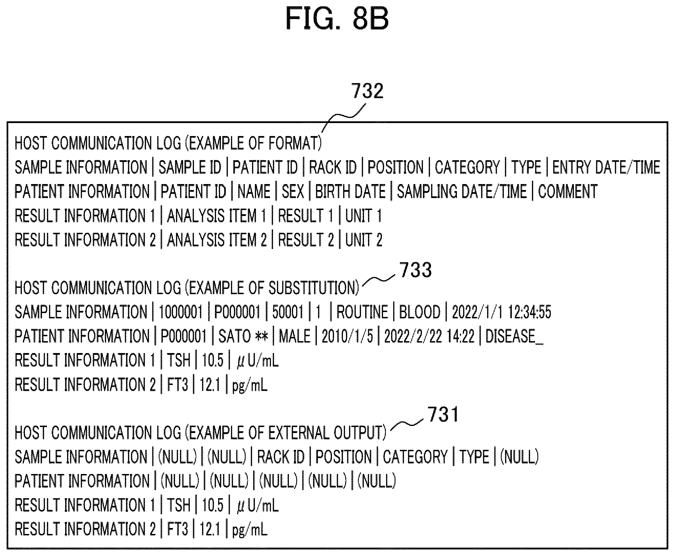

As shown in Fig. 8B, in a log (external output correspondence example) 731 of the temporary folder from which the personal information and the like are deleted, the sample ID, the patient ID, the entry date/time, the name, the sex, the birth date, the sampling date, and the comment are deletion targets.

As shown in a format example 732 shown in Fig. 8B, an output configuration of the log is determined, and for example, the log related to the patient is information including P (which means a patient record) at a predetermined position, and each piece of information is separated by a predetermined delimiter. Specifically, the log includes sample information, patient information, and one or more analysis results (result information 1 and result information 2 in the drawing). The sample information includes a sample ID, a patient ID, a rack ID, a position, sample identification information, sample type information, and entry date/time. The patient information includes a patient ID, a name, a sex, a birth date, a sampling date/time, and a comment. The analysis result includes an analysis item, an analysis result, and a unit.

A specific example is shown in substitution example 733 of Fig. 8B.

When the personal information and the like serving as the key information is deleted during output to the external medium, the databases DB1 to DB3 shown in Fig. 8A and the log 731 shown in Fig. 8B are obtained, and the databases DB1 to DB3 and the log 731 cannot be traced.

### First Embodiment

In the embodiment, the databases and the logs are output to the external medium so that the databases and the logs output to the external medium can be traced. In the following embodiments, descriptions similar to those of the comparative example are omitted. In the embodiment, the sample control screen 200 and the patient information screen 400 described above can also be displayed, and the configuration of the databases DB1 to DB3 other than the identifier is the same as that of the comparative example.

### <Procedure of Adding Record to Database>

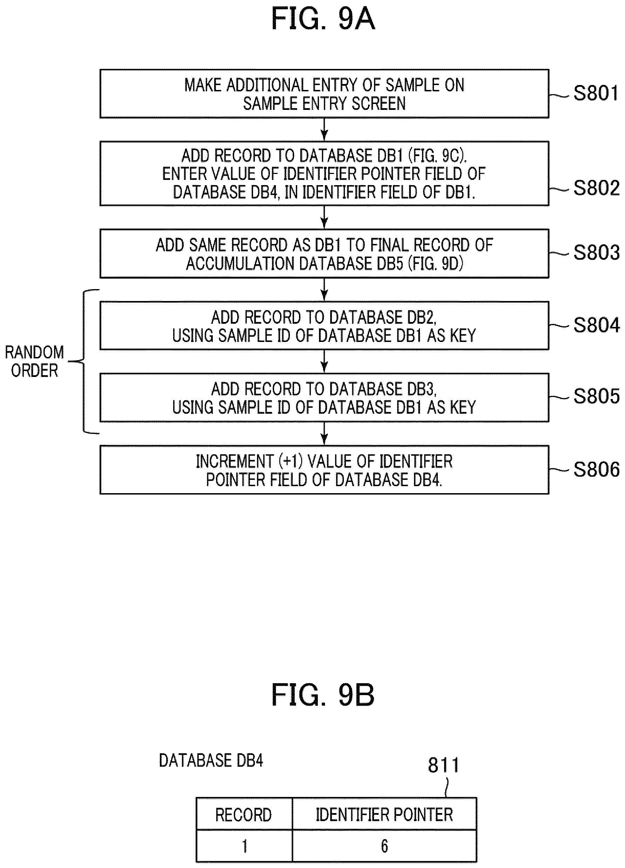

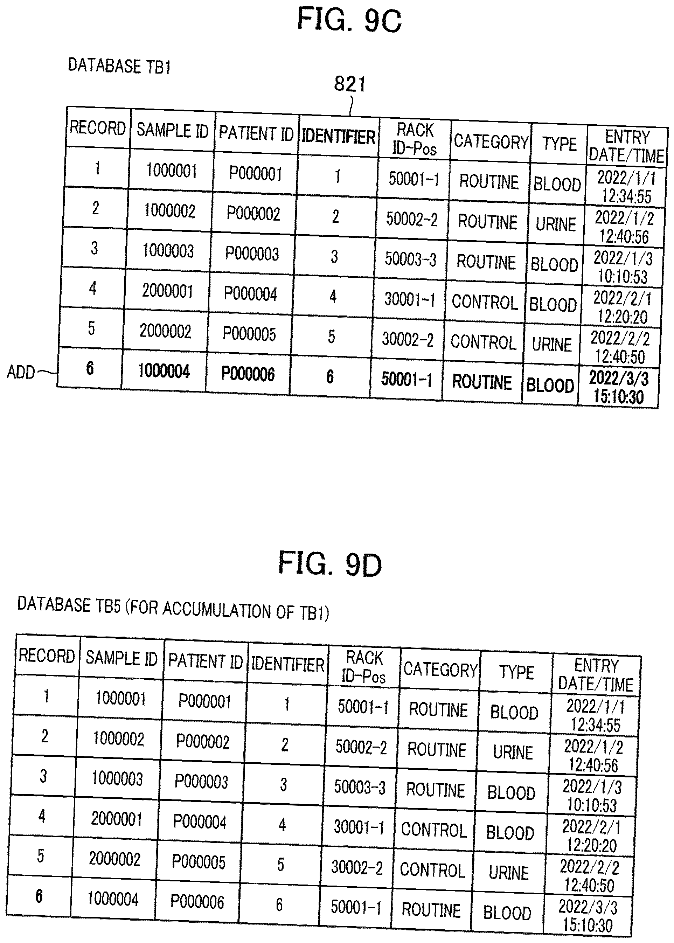

First, an operation procedure of adding a record of sample information to the database DB1 will be described with reference to Fig. 9A. When a laboratory technician or the like makes additional entry of the sample information record from the sample entry screen (not shown) displayed on the display unit 12 of the multi-item chemical analyzer 100 (step S801), the control unit 30 adds the record to the database DB1 of sample information and sets an identifier together with a sample ID (step S802). An identifier is set for each record in the database DB1. The identifier is a value stored in an identifier pointer field 811 of the database DB4 of control of identifiers shown in Fig. 9B. The control unit 30 increments a value of the identifier pointer field 811 of the database DB4 of control of identifiers every time additional entry of the sample is made. The control unit 30 uses the value of the identifier pointer field 811 for an identifier field 821 (Fig. 9C) in the database DB1 of sample information. The identifier may be implemented by alphanumeric as well as numerals, and is a unique number. For control purposes, the identifier has an incrementable configuration, such as a combination of type information and a numerical value. The identifier pointer field 811 in the database DB4 of control of identifiers is not initialized when the sample information is deleted, and an incremented value is always used for each sample entry.

Next, the control unit 30 adds the same record as the record added to the database DB1 of sample information to a final record in the database DB5 for sample information accumulation (Fig. 9D) (step S803).

Next, the control unit 30 adds records to the database DB2 of analysis results and the database DB3 of patient information using the sample ID of the database DB1 of sample information as key information (steps S804 and S805). As for the execution order of steps S804 and S805, S804 may be performed first, or S805 may be performed first.

Finally, the control unit 30 increments the value of the identifier pointer field 811 in the database DB4 of control of identifiers (step S806).

### <Procedure of Deleting Record from Database>

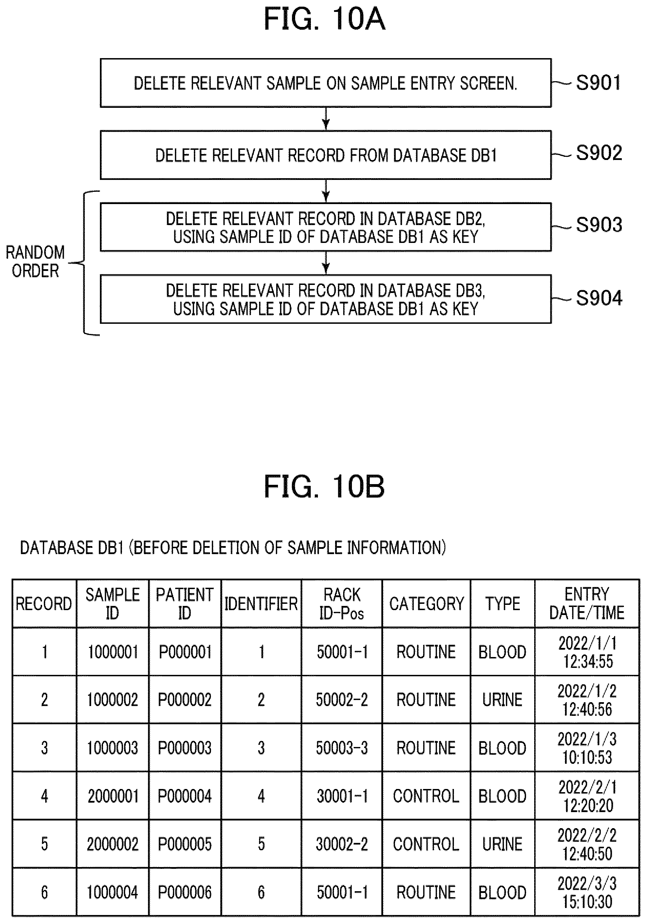

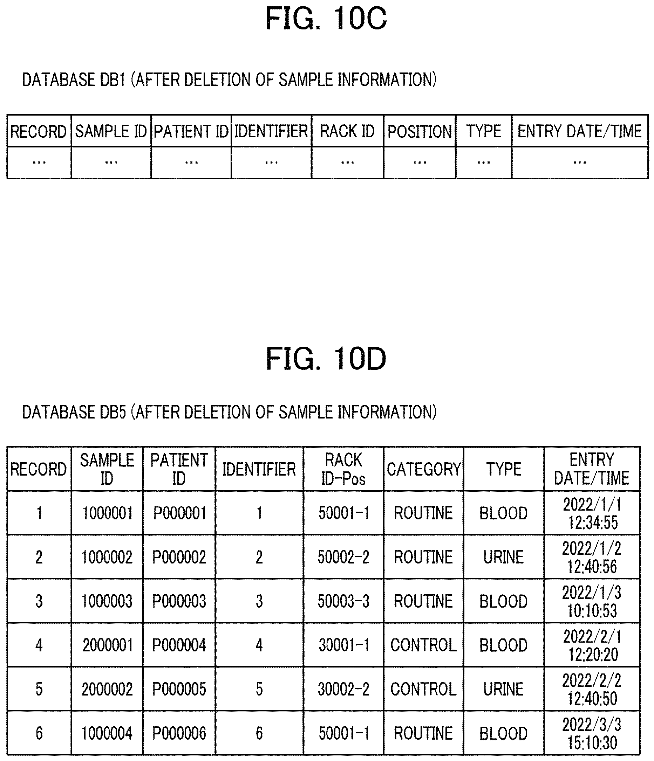

Next, an operation procedure of deleting a record of sample information from the database DB1 will be described with reference to Fig. 10A. When the laboratory technician or the like deletes the record of the sample information from the sample entry screen (not shown) displayed on the display unit 12 of the multi-item chemical analyzer 100 (step S901), the control unit 30 also similarly deletes a record of the corresponding sample information in the database DB1 of sample information (FIGS. 10B and 10C) (step S902). Therefore, even if the record of the sample information is deleted from the database DB1, a record in the database DB5 for accumulation (Fig. 10D) needs to be left, and thus the control unit 30 does not execute the operation of the record in the database DB5 for accumulation (does not delete the record).

Next, the control unit 30 deletes the records in the database DB2 of analysis results and the database DB3 of patient information using the sample ID in the database DB1 of sample information as key information (steps S903 and S904). As for the execution order of steps S903 and S904, S903 may be performed first, or S904 may be performed first.

The database DB4 of control of identifiers provides the accumulation information similarly to the database DB5 for accumulation, and therefore, the control unit 30 does not execute the operation (does not change the value of the identifier pointer field 811).

### (Modification of First Embodiment)

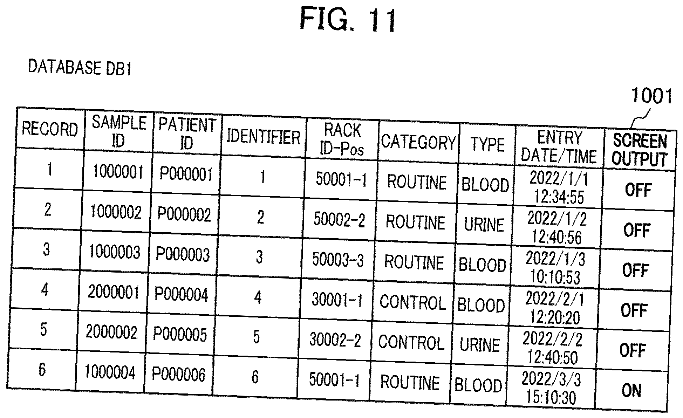

In the first embodiment, although an example in which the database DB5 for accumulation (Fig. 9D) is provided in addition to the database DB1 has been described, and a record deleted from the database DB1 to which a screen output field 1001 is added may be controlled. As shown in Fig. 11, the database DB1 of sample information of the modification of the first embodiment has the screen output field 1001. When a record of sample information is deleted, the control unit 30 sets the screen output field 1001 for the record to OFF. Only a record for which the screen output field 1001 is ON is displayed on the sample control screen 200 displayed on the display unit 12. In Fig. 11, the records of the sample information of the records 1 to 5 are deleted, and only the record of the sample information of the record 6 is displayed on the sample control screen 200. That is, in the modification of the first embodiment, a plurality of records in the database DB1 are controlled in a manner to classify the records into deleted records and non-deleted records without using the database DB5 for accumulation.

### <Method of Outputting Database and Log Information to External Medium>

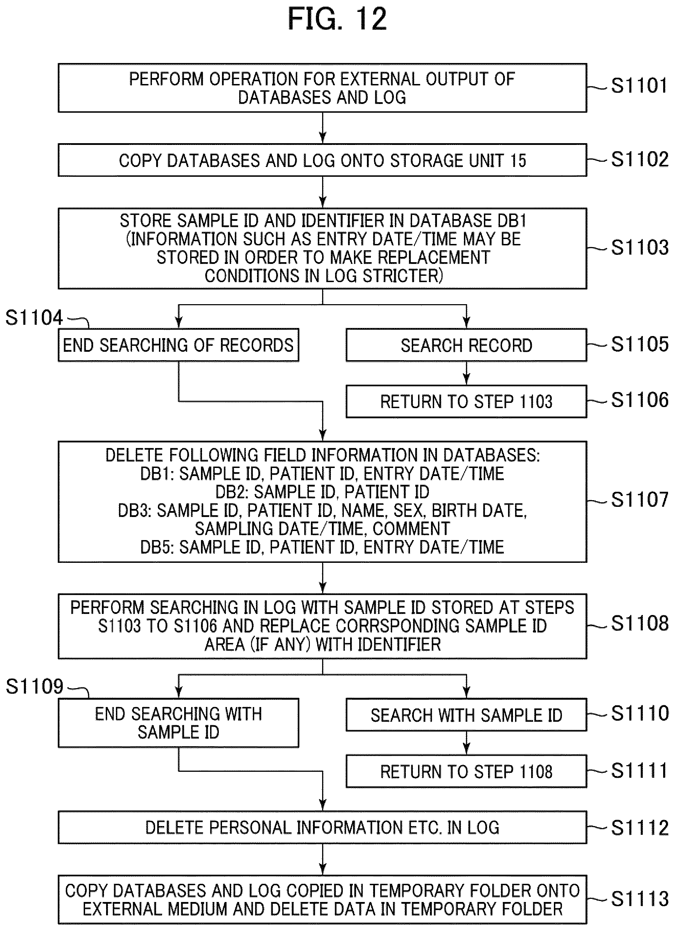

Next, a method of outputting database and log information to an external medium will be described with reference to Fig. 12. When the serviceman or the like executes an external output function of the input unit 13 to an external medium such as a USB or a DVD medium via a device screen of the display unit 12 (step S1101), the control unit 30 copies the databases DB1 to DB3, DB5, and logs stored in the storage unit 15 onto a temporary folder in the storage unit 15 (step S1102). The control unit 30 stores identifiers and sample IDs included in the database DB1 copied to the temporary folder (step S1103). The control unit 30 may store information different from the sample ID such as the entry date/time in order to make a replacement condition in the log more strict. The control unit 30 may store identifiers and sample IDs included in the database DB5.

In order to store the identifiers and the sample IDs of all the records, the control unit 30 repeatedly stores the identifiers and the sample IDs until the search for all the records is completed (steps S1104 to S1106).

Next, in order to support the GDPR, the control unit 30 deletes the sample ID, the patient ID, and the entry date/time included in the database DB1 of sample information, deletes the sample ID and the patient ID included in the database DB2 of analysis results, deletes the sample ID, the patient ID, the name, the sex, the birth date, the sampling date/time, and the comment included in the database DB3 of patient information, and deletes the sample ID, the patient ID, and the entry date/time of the database DB5 for accumulation.

Next, the control unit 30 searches the log with the sample ID stored in steps S1103 to S1106, and replaces the matched sample ID with an identifier corresponding to the sample ID (step S1108). When the search for the sample ID and the replacement of the sample ID with an identifier are completed and the sample ID search is no longer applicable, the search is performed with the next sample ID (step S1110), and the replacement with the identifier is repeatedly executed (step S1111). When the processing is executed up to the end of the record of the database DB3 of patient information, the replacement processing ends (step S1109).

When a plurality of the same sample IDs are present in the log, information such as a rack ID, a rack position, and entry date/time other than the identifier and the sample ID included in the database DB1 of sample information is stored, and the sample ID and the information such as the rack ID, the rack position, and the entry date/time in the log are also included in search conditions, so that it is possible to handle samples with the same sample ID and different identifiers.

After the replacement processing ends, the control unit 30 deletes at least personal information or the like from the log copied to the temporary folder (step S1112). Finally, the control unit 30 copies the databases and the logs copied to the temporary folder onto the external medium and deletes the databases and the logs in the temporary folder (step S1113).

### <Effects of First Embodiment>

In the first embodiment, the related information (sample ID) of the logs is replaced with the identifier set in the related information of the database DB1 which is the same as the related information of the log, the related information and the personal information (name or the like) of the databases DB1 to DB3, and the related information and the personal information of the log are deleted, and then the databases and the logs are output to the external medium. Accordingly, it is possible to trace the databases DB1 to DB3 and DB5 and the logs by using the identifiers set in the databases DB1 to DB3 and DB5 and the identifiers set in the logs.

In the first embodiment, the databases DB1 to DB3 and DB5 and the log are output to the external medium after the personal information and the related information are deleted, and therefore, the security of personal information can be strictly controlled.

In the first embodiment, the database DB5 for accumulation is provided, so that the record deleted on the sample control screen 200 can also be investigated.

In the modification of the first embodiment, the screen output field 1001 is added to the database DB1, so that the same effect as the database DB5 for accumulation can be obtained without providing the database DB5 for accumulation different from the database DB1.

### Second Embodiment

In the first embodiment, only a numeral incremented by one each time the record is added is set as the identifier, and the identifier may include characters and signs other than numerals as long as it is a unique number.

In a second embodiment, the identifier may be formed not only by a numeral but also by an alphanumeric, and is a unique number. For control purposes, the identifier has an incrementable configuration, such as a combination of sample type information and a numerical value, and is controlled in the database DB4 for identifier control.

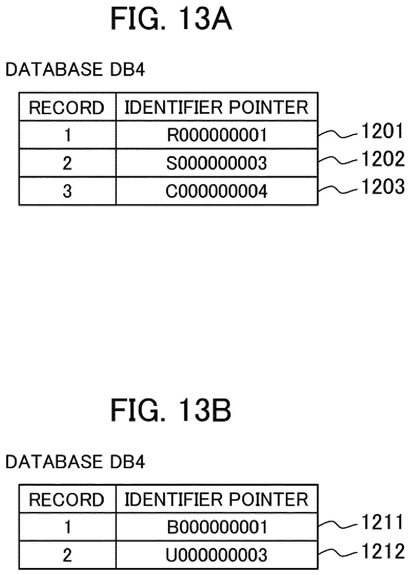

In the second embodiment, for example, sample identification information may be included in an identifier. The sample identification information included in the database DB1 of sample information (sample identification information stored in the field 204 of Fig. 3) includes Routine, Stat, Control, and the like. As shown in Fig. 13A, the alphabetic characters R, S, and C indicating the identification information are combined into an identifier, and the identifiers (1201 to 1203) are controlled for each type in the database DB4 of control of identifiers, so that the identifier is given a meaning other than tracing, and sample information conditions are narrowed to reduce the burden of investigations.

In the second embodiment, for example, the sample type information may be included in the identifier. The sample type information included in the database DB1 of sample information (the sample type information stored in the field 205 of Fig. 3) includes Blood, Urine, and the like. As shown in Fig. 13B, the alphabetic characters B and U indicating the type information are combined into an identifier, and the identifiers (1211, 1212) are controlled for each identification in the database DB4 of control of identifiers, so that the identifier is given a meaning other than tracing, and sample information conditions are narrowed to reduce the burden of investigations.

The identifier may be a combination of a plurality of pieces of information such as identification information + type information + numeral.

In a hospital where a plurality of analyzers are used, it is desirable to control identifiers by an integrated control system that centrally controls the plurality of analyzers rather than by the analyzer itself. When a unique identifier is used on the integrated control system, the centralized control can be implemented by using the database in Fig. 9B or Fig. 13A. In this case, for example, when an analyzer B uses an identifier after an analyzer A uses an identifier 1, the analyzer B uses an identifier 2 next. That is, the identifier pointer field information in the identifier control database DB5 is shared and used between different analyzers.

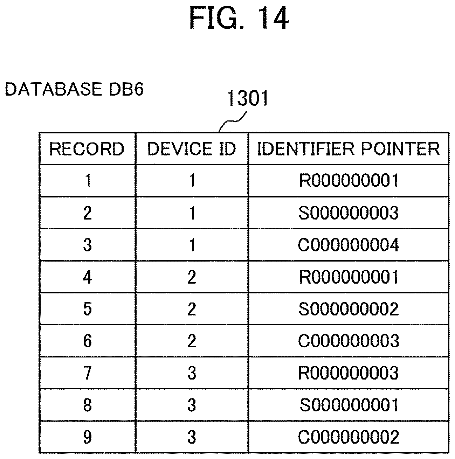

Alternatively, when a number is assigned to each analyzer, a device ID field 1301 is provided in a database DB6 for control of identifiers as shown in Fig. 14, and the device ID is handled as key information between the integrated control system and the analyzer, thereby enabling control of each analyzer. The device ID includes numerical values or characters, and uses a device ID common to the integrated control system and the analyzer.

### Third Embodiment

PTL 1 (JP2014-062917A) discloses a technique of differentiating pieces of information to be displayed in a user mode including sample information and a service mode not including sample information. In PTL 1 (JP2014-062917A), the display of the sample ID is permitted in the service mode. However, the display of the sample ID should not be permitted because the sample ID is related information linked to the personal information.

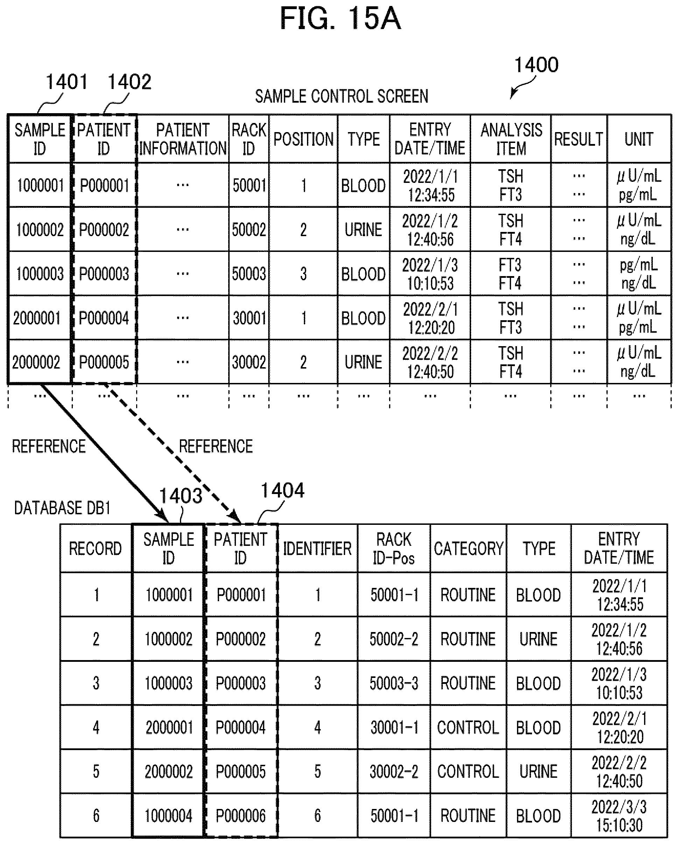

Fig. 15A shows a reference status of a sample control screen 1400 and the sample information database DB1 in a third embodiment. In the user mode (first display mode), an area 1401 for displaying a sample ID and an area 1402 for displaying a patient ID on the sample control screen 1400 are displayed with reference to a sample ID field 1403 and a patient ID field 1404 of the database DB1 of sample information, respectively.

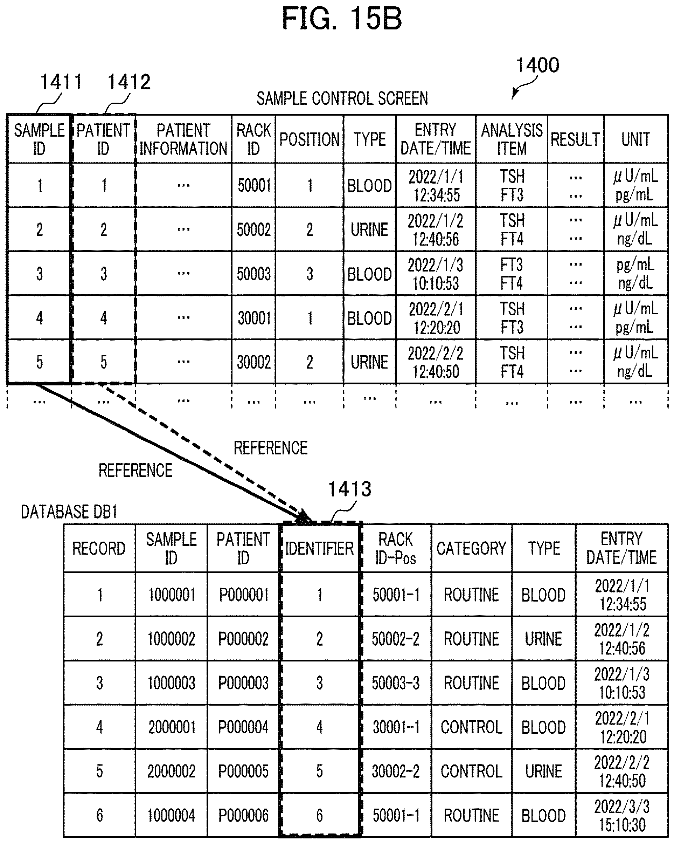

When switching to the service mode (second display mode), as shown in Fig. 15B, each of reference destinations of an area 1411 for displaying a sample ID and an area 1412 for displaying a patient ID on the sample control screen 1400 is changed to an identifier field 1413 of the database DB1 of sample information. Therefore, an identifier can be displayed on the area 1411 for displaying a sample ID and the area 1412 for displaying a patient ID on the sample control screen 1400.

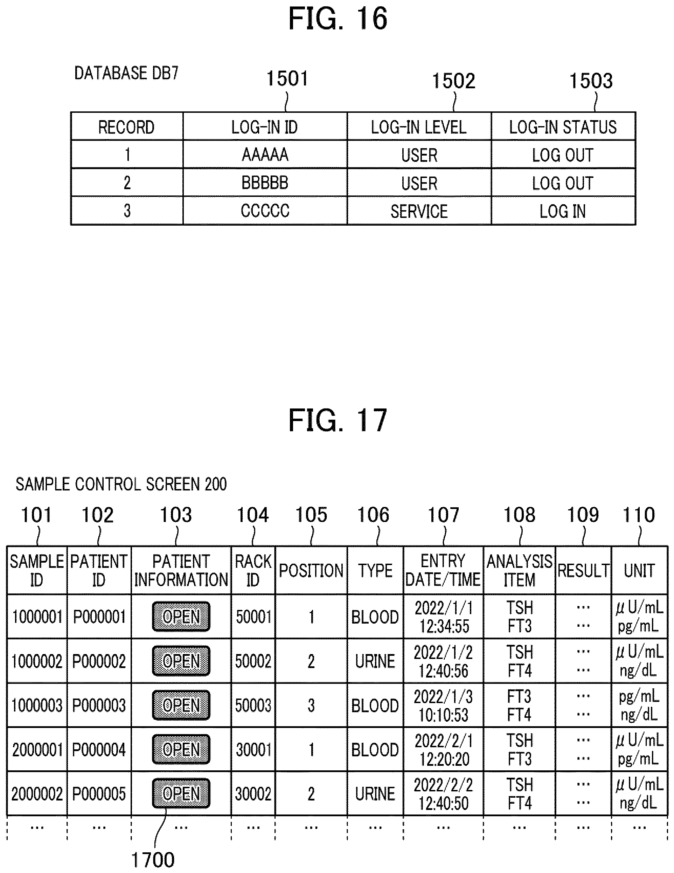

As shown in Figs. 16 and 17, a button 1700 for opening the patient information screen 400 (Fig. 5) determines validity by referring to a log-in level field 1502 of a database DB7 for controlling a log-in ID in Fig. 16. In the case of the service mode, as shown in Fig. 17, the button 1700 for opening the patient information screen 400 is set to inactive (non-display or grayed out) to prohibit reference to the patient information screen 400. The database DB7 that manages the log-in information includes a log-in ID field 1501, the log-in level field 1502, and a log-in status field 1503.

### <Effects of Third Embodiment>

The sample control screen 1400 can be provided in a state in which the personal information or the like is hidden to the serviceman or the like by providing the service mode in which a part of the information is hidden. Therefore, the personal information can be prevented from leaking to the serviceman or the like.

The patient information screen 400 including personal information and the like can be prevented from being displayed by inactivating the button 1700 in the service mode.

### <Modification>

The invention is not limited to the above embodiments and includes various modifications. For example, the above embodiments have been described in detail to facilitate understanding of the invention, and the invention is not necessarily limited to those including all the configurations described above. A part of a configuration of a certain embodiment can be replaced with a configuration of another embodiment, and a configuration of another embodiment can also be added to a configuration of a certain embodiment. In addition, another configuration can be added to a part of a configuration of each embodiment, and the part of the configuration of each embodiment can be deleted or replaced with another configuration.

For example, in the above-described embodiment, an example in which a sample ID of a log is replaced with an identifier has been described, and the identifier set in the database DB1 which is the same as the sample ID may be set to the sample ID of the log, and the personal information such as the sample ID may be deleted when output to the external medium.

The databases and the logs are stored in the storage unit 15 in the above-described embodiment, and the databases and the logs may be stored in a plurality of storage units (a local storage unit and a storage unit on a cloud) in a distributed manner.

In the first embodiment described above, the database DB5 for accumulation is used, and in the modification of the first embodiment, the database DB1 having the screen output field 1001 is used. However, the database configuration is not limited to these. For example, the database DB1 and a database for deletion may be used. When a record is added, the record is added to the database DB1, and no record is added to the database for deletion. When a record is deleted, the record is deleted from the database DB1, and the deleted record is added to the database for deletion. Accordingly, the same effects as those of the first embodiment and the modification of the first embodiment can be obtained.

In the third embodiment, although the display mode is switched according to a log-in level of a log-in user, a method of switching the display mode is not limited to the switching method according to the log-in level, and the display mode may be switched by, for example, a manual operation of a laboratory technician or the like.

### Reference Signs List

1: sample container,
2: sample disk,
3: processor,
4: interface,
5: sample dispensing mechanism,
6, 9: liquid level detector,
7: reagent dispensing mechanism,
8: reaction disk,
10: reagent container,
11: reaction container,
12: display unit,
13: input unit,
14: reagent ID reader,
15: storage unit,
16: memory,
17: reagent disk,
18: printer,
19: A/D converter,
20: reader,
21: information control system,
22: host computer,
23: I/O device,
24: reader,
30: control unit,
40: network,
50: analyzing unit,
101, 401, 1401, 1411: area for showing a sample ID,
102, 1402, 1412: area for showing a patient ID,
103: area for showing a link to patient information details,
104: area for showing a rack ID,
105: area for showing a position in a rack,
106: area for showing sample type information,
107: area for showing the entry date/time of a sample,
108: area for showing an analysis item,
109: area for showing an analysis result,
110: area for showing the unit,
200, 1400: sample control screen,
201, 301, 501, 701, 711, 721, 1403: sample ID field,
202, 302, 502, 702, 712, 722, 1404: patient ID field,
203: rack ID-Pos field,
204: identification information field,
205: type information field,
206, 706: entry date/time field,
303: analysis item field,
304: analysis result field,
305: unit field,
400: patient information screen,
402: area for patient information, etc.
503, 723: name field
504, 724: sex field,
505, 725: birth date field,
506, 726: sampling date/time field,
507, 727: comment field,
731: log (example of external output),
732: example of format,
733: example of substitution,
811: identifier pointer field,
821, 1413: identifier field,
1001: screen output field,
1201, 1202, 1203, 1211, 1212: identifier,
1301: device ID field,
1501: log-in ID field,
1502: log-in level field,
1503: log-in status field
1700: button for opening the patient information screen

## Claims

1. An analyzer comprising:
an analyzing unit that analyzes a sample;
a storage unit that stores a database in which related information linked to personal information of a patient having supplied the sample, and sample information of the sample to be analyzed by the analyzing unit, an analysis result of the sample by the analyzing unit, or the personal information of the patient are stored in a corresponding manner and a log including the related information; and
a control unit that
sets a unique identifier for each record of the database,
sets, for the related information of the log, the identifier set for the related information of the database which is the same as the related information of the log, and
after deleting the related information of the database and the related information of the log, outputs the database and the log to an external device.

2. The analyzer according to Claim 1, wherein, by replacing the related information of the log with the identifier set for the related information of the database which is the same as the related information of the log, the control unit sets, for the related information of the log, the identifier set for the related information of the database which is the same as the related information of the log.

3. The analyzer according to Claim 1, wherein
the control unit further deletes the personal information of the patient in the database and outputs the database and the log to the external device.

4. The analyzer according to Claim 1, wherein
the storage unit further stores a database for accumulation in which the analysis result of the sample by the analyzing unit and the related information are stored in a corresponding manner, and
the control unit,
when a record is to be added to the database, adds a record corresponding to the record to the database for accumulation, and
when the record is to be deleted from the database, keeps the record corresponding to the record in the database for accumulation.

5. The analyzer according to Claim 1, wherein
the control unit controls a plurality of records in the database in a manner to classify the records into records to be displayed on a display unit and records not to be displayed on the display unit.

6. The analyzer according to Claim 1, wherein
the identifier includes a character or sign indicating a type of the sample.

7. The analyzer according to Claim 1, wherein
the control unit,
in a first display mode, displays information including the related information on a display unit and
in a second display mode, displays information except the related information on the display unit.

8. The analyzer according to Claim 7, wherein
the control unit,
in the first display mode, displays on the display unit a button to show the personal information, and shows the personal information on the display unit according to selection with the button, and
in the second display mode, prohibits reference to the personal information, or inactivates the button.

9. An information output method comprising:
storing, in a storage unit, a database in which related information linked to personal information of a patient having supplied a sample, and sample information of the sample to be analyzed by an analyzing unit for analyzing the sample, an analysis result of the sample by the analyzing unit, or the personal information of the patient are stored in a corresponding manner, and a log including the related information;
setting a unique identifier for each record of the database,
setting, for the related information of the log, the identifier set for the related information of the database which is the same as the related information of the log, and
after deleting the related information of the database and the related information of the log, outputting the database and the log to an external device.

10. The information output method according to Claim 9, wherein
setting the identifier is replacing the related information of the log with the identifier set for the related information of the database which is the same as the related information of the log.

11. The information output method according to Claim 9, wherein
outputting the database and the log includes outputting the database and the log to the external device after deleting the related information and the personal information of the database, and the related information and the personal information of the log.

12. The information output method according to Claim 9, further comprising:
storing a database for accumulation in which the analysis result of the sample by the analyzing unit and the related information are stored in a corresponding manner, in the storage unit;
when a record is to be added to the database, adding a record corresponding to the record to the database for accumulation, and
when the record is to be deleted from the database, keeping the record corresponding to the record in the database for accumulation.

13. The information output method according to Claim 9, further comprising:
controlling a plurality of records in the database in a manner to classify the records into records to be displayed on a display unit and records not to be displayed on the display unit.

14. The information output method according to Claim 9, wherein
the identifier includes a character or sign indicating a type of the sample.

15. The information output method according to Claim 9, further comprising:
in a first display mode, displaying information including the related information on a display unit, and
in a second display mode, displaying information except the related information on the display unit.

16. The information output method according to Claim 15, further comprising:
in the first display mode, displaying on the display unit a button to show the personal information, and showing the personal information on the display unit according to selection with the button, and
in the second display mode, prohibiting reference to the personal information, or inactivating the button.
